# EUROPEAN PATENT SPECIFICATION

(11) **EP 0 598 396 B1**
(45) Date of publication and mention of the grant of the patent: **02.09.1998**
(21) Application number: 93118542.5
(22) Date of filing: 18.11.1993
(51) Int. Cl.: C07D 213/73, C07D 213/76

(54) **Improved method for the preparation of 3-Amino-2-chloro-4-alkylpyridines**
Verbesserte Methode zur Herstellung von 4-Alkyl-3-Amino-2-Chlor-Pyridinen
Méthode améliorée pour la préparation de 4-alkyle-3-amino-2-chloro-pyridines

(30) Priority: 18.11.1992 US 977962
(43) Date of publication of application: 25.05.1994
(73) Proprietor: BOEHRINGER INGELHEIM PHARMACEUTICALS INC., Ridgefield, Connecticut 06877-0368 (US)
(72) Inventor: Nummy, Laurence John, Newburgh, New York 12550 (US)
(74) Representative: Laudien, Dieter, Dr.

(56) References cited:
- EP-A- 0 136 995
- EP-A- 0 143 768
- EP-A- 0 429 987
- WO-A-92/22531
- WO-A-92/22532
- US-A- 4 172 095
- US-A- 4 209 464
- JOURNAL OF THE AMERICAN CHEMICAL SOCIETY vol. 95, no. 2 , 24 January 1973 pages 588 - 589 GASSMAN PG & GRUETZMACHER G 'Specific ortho alkylation of aromatic amines'
- JOURNAL OF THE AMERICAN CHEMICAL SOCIETY vol. 95, no. 13 , 27 June 1973 pages 4453 - 4455 GASSMAN PG & HUANG CT 'Specific ortho substitution of aromatic heterocyclic amines. Conversions of 2-aminopyridines'
- JOURNAL OF THE CHEMICAL SOCIETY, CHEMICAL COMMUNICATIONS 1974 pages 685 - 686 GASSMAN PG & HUANG CT 'Selective formylation of 2-aminopyridines'

## Description

### FIELD OF THE INVENTION

This invention relates to a novel method for preparing certain 3-amino-2-chloro-4-alkylpyridines.

### BACKGROUND OF THE INVENTION

U.S. Patent Application Serial No. 600,390, filed October 19, 1990, (= EP-A-429987) entitled "5,11-Dihydro-6H-Dipyrido[3,2-b:2',3'-e][1,4]Diazepines and Their Use in the Prevention or Treatment of HIV Infection", describes novel 5,11-dihydro-6H-dipyrido[3,2-b:2',3'-e][1,4]diazepines useful in the prevention and treatment of HIV infection and methods or preparing these compounds.

3-Amino-2-chloro-4-alkylpyridines are useful intermediates in the preparation of 4-alkyl-5,11-dihydro-6H-dipyrido[3,2-b:2',3'-e][1,4]diazepines.

Processes suitable for the preparation of 3-amino-2-chloro-4-alkylpyridines (I) and of the starting material of formula IV are known in the art. J. Am. Chem. Soc. (1973) 95, 4453-4455; US-A-4 172 095 and US-A-4 209 464 disclose reductive conversions of substituted phenyls or substituted pyridines using Raney nickel.
J. Am. Chem. Soc. (1973) 95, 588-589; J. Am. Chem. Soc. (1973) 95, 4453-4455; J. Chem. Soc., Chem. Commun. (1974) 685-686; US-A-4 172 095; US-A-4 209 464; EP-A-0 136 995 and EP-A-0 143 768 disclose various ways to rearrange sulfilimines.
The purpose of the invention is to provide alternative advantageous routes.

### SUMMARY OF THE INVENTION

3-Amino-2-chloro-4-alkylpyridines prepared by the novel process of this invention have the formula: 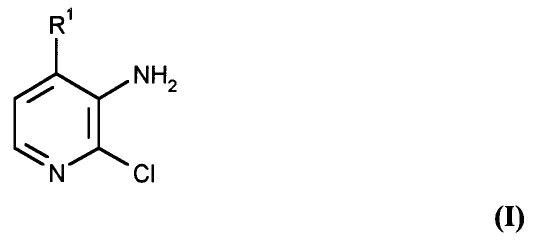 wherein R¹ is a linear, branched or cyclic hydrocarbon of from one to eight carbon atoms, optionally substituted with one or more electron stabilizing groups.

The process for this invention for the preparation of the compound of formula I is outlined below:

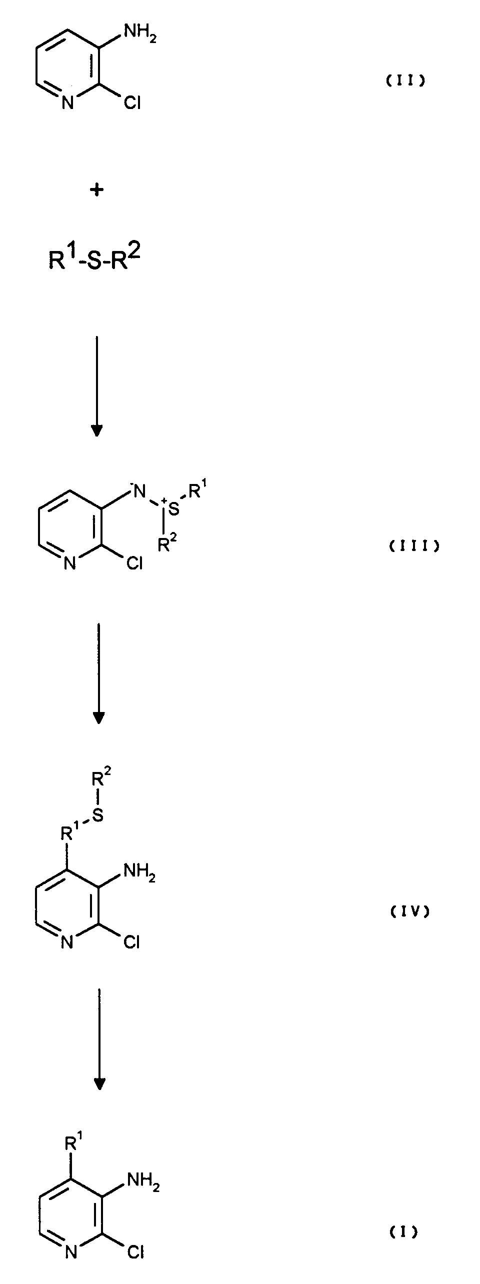

### DETAILED DESCRIPTION OF THE INVENTION

The raw materials used in the processes described below are available in commercial quantities.
The above reaction scheme outlines the preferred sequence of 3 reactions for the preparation of compounds of formula I. If these reactions are combined the process is operationally straight forward to carry out (three steps and a final fractional distillation to purify the product) on a large scale. However, it is also possible to combine the individual steps described herein with suitable reaction steps known in the art. For example step 3 described below may be combined with a known process for the preparation of compounds IV.

Step 1: reacting a compound having the formula 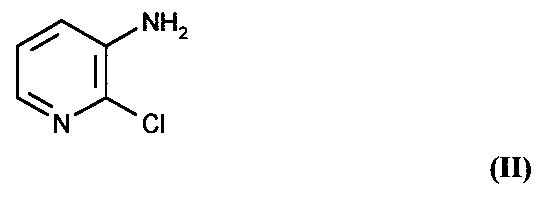 with a compound of the formula R¹-S-R², wherein
(a) R¹ and R² are each a linear, branched or cyclic hydrocarbon of from one to eight carbon atoms; or
(b) R¹ is a linear, branched or cyclic hydrocarbon of from one to eight carbon atoms, and R² is an aromatic or heteroaromatic group, such as phenyl, naphthyl, benzothiazolyl, benzoxazolyl, benzimidazolyl, pyridyl, furyl or thienyl; or
(c) R¹ is a linear, branched or cyclic hydrocarbon of from one to eight carbon atoms, and R² is a linear, branched or cyclic hydrocarbon of from one to eight carbon atoms, wherein the carbon atom in R² bonded to the sulfur atom has no hydrogen bonded to it; or
(d) R² is a linear, branched or cyclic hydrocarbon of from one to eight carbon atoms, and R¹ is a linear, branched or cyclic hydrocarbon of from one to eight carbon atoms optionally substituted with one or more electron stabilizing groups separated from the sulfur atom by one saturated carbon atom substituted with at least one hydrogen atom; or
(e) R¹ and R² are each a linear or branched hydrocarbon of from one to eight carbon atoms, joined together with the sulfur atom to form a ring of from 5 to 8 members, wherein the ring can optionally be substituted with one or more electron stabilizing groups separated from the sulfur atom by one saturated carbon atom substituted with at least one hydrogen atom,
in the presence of an halogenating agent such as N-chlorosuccinimide, sulfurylchloride, chloramine-T, trichloroisocyanurate, chlorine, calcium hypochlorite, t-butyl hypochlorite, at a temperature ranging from -60°C to 25°C, for 1 to 24 hours, to produce a compound of the formula: 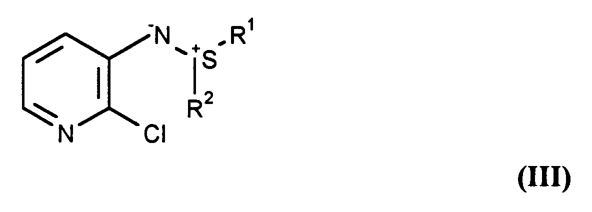 Examples of suitable electron stabilizing groups are C₆-C₁₀ aryl, C₁-C₈ carboalkoxy, carboxyamido, cyano, C₁-C₁₀ acyl (derived from aromatic or alkyl carboxylic acids), nitro, C₁-C₈ alkoxy, phosphoryl, C₁-C₈ alkyl, or C₆-C₁₀ arylthio, arylsulfinyl or arylsulfonyl.

Preferably, R¹ and R² are each a linear, branched or cyclic hydrocarbon of from one to eight carbon atoms.

More preferably, R¹ is a linear hydrocarbon of from one to four carbons and R² is a linear, branched or cyclic hydrocarbon of from one to eight carbon atoms.

Most preferably, R¹ is methyl or ethyl and R² is methyl, phenyl or 2-benzothiazolyl.

Step 2: heating the compound produced in Step 1 in the presence of (i) triethylamine, methanesulfonic acid, and an organic solvent such as toluene; or (ii) an organic solvent such as toluene, and a phenol of the formula: 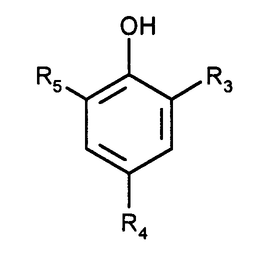
wherein R₃, R₄ and R₅ can each be H, C₁-C₈ straight chain or branched alkyl, Cl, Br or CH₂SR_{2,} wherein R₂ is as defined above;
R₄ can be -C(CH₃)₂-A, 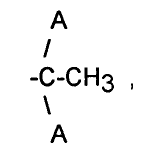 OR_{6,} wherein R₆ is C₁-C₈ straight chain alkyl, -[CH₂CH₂0]ₙ-CH₂CH₂OH and n is a number from 0 to 20, and A has the formula 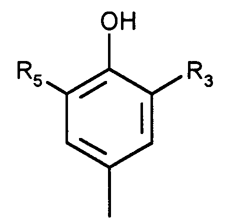
R₅ can be -CH₂-B, wherein B has the formula 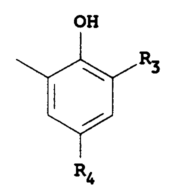
wherein R₃ is as defined above and R₄ can be as defined above, provided that it does not contain the group A,
or a phenol of the formula 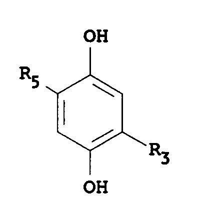
wherein R₃ is as defined above and R₅ is H or R_{3,}
   at a temperature ranging from 60°C to 110°C, for 3 to 24 hours, to produce a compound of the formula

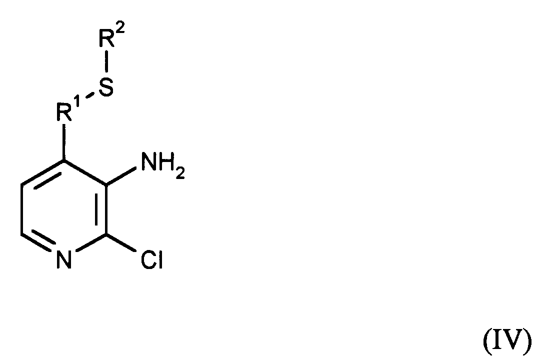

Preferably, the phenol is selected from the group consisting of 2-6-di-t-butyl-4-methylphenol, 2-t-butyl-4-methylphenol or 2-t-butyl-4-methyl-6-methylthiomethylphenol.

Most preferably, the phenol is 2-t-butyl-4-methylphenol (BHT).

Step 3: alkylating the compound produced in step 2 in the presence of an alkylating agent, at a temperature ranging from 20°C to 100°C, for 1 to 140 hours, to produce a sulfonium salt and then contacting the sulfonium salt so produced with zinc at a temperature of 0°C to 50°C, for 1 to 24 hours, to produce the compound of formula I.

Preferably, the alkylating agent is selected from the group consisting of methyl iodide, dimethylsulfate, allyl chloride, benzyl chloride, benzyl bromide, methyl chloride or chloroacetic acid.

Most preferably, the alkylating agent is dimethylsulfate.

Example 1 illustrates the preparation of the 3-amino-2-chloro-4-alkylpyridines of formula I.

### EXAMPLE 1

### PREPARATION OF 3-AMINO-2-CHLORO-4-METHYLPYRIDINE

### A) PREPARATION OF S,S-DIMETHYL-N-(3-PYRIDYL)SULFILIMINE

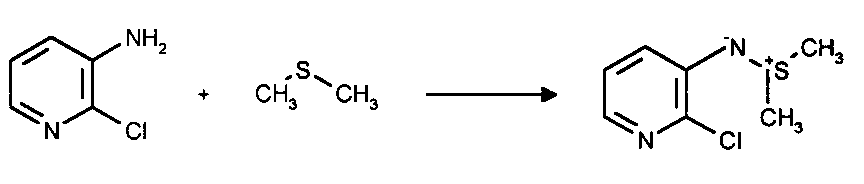

Prior to use, all glassware was dried in an oven at 105°C and cooled either in a dessiccator or by venting to a nitrogen atmosphere while cooling. Dichloromethane was dried by storage over activated 4A molecular sieves.

A 3000 ml three-neck round bottom flask equipped with overhead mechanical stirrer and nitrogen inlet stopcock was charged with 3-amino-2-chloropyridine, 44.27g (0.34 moles). The apparatus was maintained under a slight positive pressure of nitrogen until the work up. Dichloromethane, 347 ml, dried by standing over activated 4A molecular sieves, was then introduced to the flask. Stirring was initiated and the flask was cooled until the internal temperature was -28°C, to produce a first slurry. Methyl sulfide, 25.68g (0.41 moles) was then added to the slurry via syringe over two minutes. The internal temperature of the flask was then allowed to equilibrate at -20°C.

A solution of N-chlorosuccinimide was prepared as follows: A 1000 ml round bottom flask equipped with magnetic stir bar, was charged with N-chlorosuccinimide, 45.98g (0.34 moles), followed by dichloromethane, 950 ml. This flask was sealed with a rubber septum and purged with nitrogen to produce a second slurry. The second slurry was stirred for 1 hour and 20 minutes, producing a cloudy solution.

The cloudy solution of N-chlorosuccinimide was added dropwise to the first slurry for 1 hour and 50 minutes, to produce a first reaction mixture. The temperature of the reaction mixture was maintained at -20°C. During the addition of N-chlorosuccinimide, the reaction mixture first became homogeneous and then a precipitate formed. When the addition of N-chlorosuccinimide was complete, the reaction mixture was allowed to stir at -20°C for an additional 22 hours. The reaction mixture was then quenched by transferring it, over 15 minutes, to a stirred solution of 10% aqueous sodium hydroxide, 995 ml, which had been previously cooled to -5°C, to produce a second reaction mixture. During the transfer of the first reaction mixture, the temperature of the second reaction mixture was maintained between 0°C and -5°C. When the transfer of the first reaction mixture was complete, the second reaction mixture was stirred for an additional 10 minutes and then allowed to settle, to produce an aqueous layer and an organic layer. The aqueous layer and organic layer were separated. The aqueous layer was then extracted (while still cold) with two successive 250 ml volumes of dichloromethane. The two extracts were then dried over anhydrous sodium sulfate, 100g, filtered and then concentrated in vacuo, to give 62.15g of an off-white colored solid. The solid so produced was slurried with toluene, 50 ml, at ambient temperature and the resultant supernatant so formed was then removed. This step was repeated twice with 25 ml aliquots of toluene. Residual toluene was removed by rinsing the solid with hexane. The resultant solid was finally dried at 30°C under vacuum. This provided 52.09g of S,S-dimethyl-N-(3-pyridyl)sulfilimine (80% yield).

The resultant solid was characterized by combustion analysis:

| | | |
|---|---|---|
| %C | Calculated 44.56 | Found 44.53 |
| %H | Calculated 4.81 | Found 4.78 |
| %N | Calculated 14.85 | Found 14.83 |
| %Cl | Calculated 18.79 | Found 19.09 |
| %S | Calculated 16.99 | Found 17.03 |

Proton NMR:
   60 MHz; CDCl₃; ppm downfield shift from TMS ref: 2.75, (s), 6H; 7.2, (m), 2H; 7.8, (m), 1H
IR:
   KBr pellet cm⁻¹; 3093-3001, 2910, 1560-1442 (Pyridine ring stretching), 1388, 954 and 917 (S-methyl deformation), 900 (N-S stretch), 778, 688, 731
Melting point: 107-112°C with decomposition (uncorrected)

### B) PREPARATION OF 3-AMINO-2-CHLORO-4-METHYLTHIOMETHYLPYRIDINE

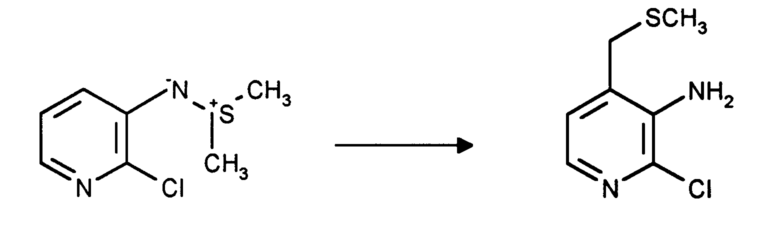

Prior to use, all glassware was dried in an oven at 105°C and cooled either in a dessiccator or by venting to a nitrogen atmosphere while cooling. Toluene and triethylamine were also dried by storage over activated 4A molecular sieves.
i) A 100 ml three-neck round bottom flask equipped with a magnetic stir bar, reflux condenser and thermocouple, was charged with the S,S-dimethyl-N-(3-pyridyl)sulfilimine prepared in A (4.0g, 21.2 mmoles) and 21 ml of toluene. The flask was purged with nitrogen and maintained under inert atmosphere. 14.6 ml (106 mmoles) of triethylamine was added to the flask with stirring over 5 minutes, to produce a reaction mixture. The reaction mixture was then heated to 85°C for 23 hours. 1.4 ml (21.2 mmoles) of methanesulfonic acid was then added dropwise to the reaction mixture over 7 minutes, to produce a second reaction mixture. The second reaction mixture was heated at 85-90°C for the remainder of 2 hours. The second reaction mixture was then allowed to cool to 25°C. 50 ml of water was then added to reaction mixture and stirred for 10 minutes, to produce an aqueous layer and a dark toluene layer. The aqueous layer is drained off and the dark toluene layer is extracted twice with 25 ml portions of water. The resultant dark toluene layer is then concentrated in vacuo to provide 3.2g of a dark oil. The oil is purified using silica gel flash chromatography (eluent: 6% ethyl acetate in dichloromethane (v/v)) to produce 2.2g (55%) of 3-amino-2-chloro-4-methylthiomethylpyridine (R_{f}=0.43).
The product so produced was characterized as follows:
Low Resolution CIMS:
CH₄; m/z 153 (MH⁺-HCl), m/z 141 (MH⁺-CH₃SH)
Proton NMR:
270 MHz, CDCl₃, ppm downfield shift from TMS ref: 1.97, (s), 3H; 3.64, (s), 2H; 4.67, (brs), 2H; 6.92, (d, J=4.76 Hz), 1H; 7.73, (d, J=4.76 Hz), 1H. N.O.E. enhancements SCH₃ at 1.97 ppm and NH₂ at 4.67 ppm; CH₂S at 3.64 ppm and NH₂ at 4.67 ppm.
¹³C NMR:
68 MHz, CDCl₃, ppm downfield shift from TMS ref: 14.305, 34.029, 123.994, 130.172, 137.023, 137.472, 138.027
High Resolution MS:

| | | |
|---|---|---|
| C₇H₉N₂³⁵CIS: | Calculated 188.01749 | Found 188.0170 |
| C₇H₉N₂³⁷CIS: | Calculated 190.01455 | Found 190.0141 |

ii) A 1000 ml three-neck round bottom flask equipped with mechanical stirrer, reflux condenser and thermocouple, was charged with 100g (530 mmoles) of the S,S-dimethyl-N-(3-pyridyl)sulfilimine prepared in A and 300 ml of toluene. 4.353g (26.5 mmoles) of 2-t-butyl-4-methylphenol and an additional 20 ml of toluene, were then added, to produce a reaction mixture. The flask was purged with nitrogen and maintained under inert atmosphere. The reaction mixture was heated to 85-95°C for 5 hours with stirring and then allowed to cool to ambient temperature. The dark toluene solution so produced is decanted from the tar and the decanted solution was then concentrated under reduced pressure to produce a dark oil weighing 98.26g. The yield of 3-amino-2-chloro-4-methylthiomethylpyridine was determined to be 68% by NMR analysis using trichloroethylene as internal standard. The crude product could be desulfurized without further purification.

### C) PREPARATION OF 3-AMINO-2-CHLORO-4-METHYLPYRIDINE

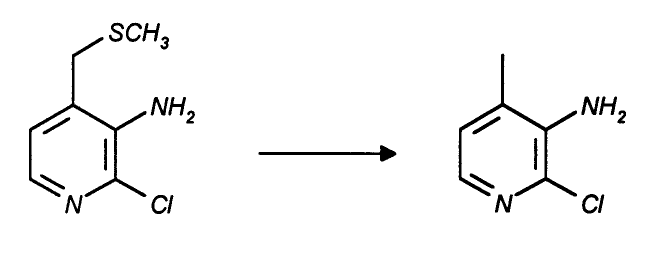

(i) A 12-liter three neck round bottom flask was equipped with mechanical overhead stirrer, inlet stopcock (tee-connected to nitrogen supply and oil bubbler vent) and type K thermocouple probe, to produce a reaction vessel. The reaction vessel was purged and maintained under nitrogen atmosphere. Grace 4200 Raney nickel, 2.71 Kg (dry basis), was charged to the vessel as a slurry in water. The resultant supernatant liquid in the vessel was transferred out of the vessel via 12 gauge line using nitrogen pressure, leaving a residue in the vessel. Methanol, 2.38 liters, was charged to the residue in the vessel and the resulting reaction mixture was stirred for 20 minutes. The stirring was terminated and the nickel in the reaction mixture was allowed to settle for 30 minutes. After the nickel settled, the resultant supernatant liquid was then transferred out of the vessel via 12 gauge line using nitrogen pressure. Stirring was resumed and a methanol solution (19.8% w/w) of crude 3-amino-2-chloro-4-methylthiomethylpyridine prepared as described in B above, was added over a one hour period via transfer line using nitrogen pressure, to produce a second reaction mixture. The internal temperature of the second reaction was maintained below 40°C using a cool water bath. Fifty minutes after addition of the sulfide, TLC analysis of the second reaction mixture revealed that the desulfurization was complete. The second reaction mixture was then gently heated and a final reaction temperature of 51°C was reached 1 hour 25 minutes after addition of sulfide. The second reaction mixture was then allowed to cool to ambient temperature overnight. Stirring was then discontinued, producing a reaction solution and a nickel residue. The reaction solution is transferred out of the vessel via 12 gauge line using nitrogen pressure. The nickel residue was extracted three times, each time with 2.4 liters of fresh methanol, stirring for twenty minutes and then settling for one hour. The combined extracts were concentrated under reduced pressure and a maximum temperature of up to 65°C, producing a dark oil weighing 330 grams. The oil was taken up into 2.5 liters of toluene, filtered and concentrated again under pressure up to 65°C, producing a residue weighing 307 grams. This residue was purified by fractional destillation. The resultant pure distilled 3-amino-2-chloro-4-methylpyridine was characterized as follows:
Boiling point: 140°C/1333 Pa ("10 mmHg")
Melting point: 66-68°C (uncorrected)
Combustion analysis:

| | | | | |
|---|---|---|---|---|
| Calculated % C, H, N, Cl: | 50.54, | 4.95, | 19.65, | 24.86 |
| Found % C, H, N, Cl: | 50.54, | 4.90, | 19.73, | 24.60 |

NMR:
250 MHz proton spectrum; CDCl₃ solvent; ppm downfield shift from TMS reference: 2.178, singlet, 3H; 4.173, br. singlet, 2H; 6.899, doublet J=4.76 Hz; 1H; 7.665, doublet J=4.74 Hz, 1H
68 MHz ¹³C spectrum; CDCl₃ solvent; ppm downfield shift (from 0.00 with ¹³CDCl₃ reference at 76.996 ppm): 17.093, 124.49, 131.665, 136.302, 137.476, 138.099
Mass spectrum:
low resolution CI; methane; m/z, intensity: 143, 100; 145, 31.92
ii) 3-amino-2-chloro-4-methylthiomethylpyridine prepared above in B (90.2 mg, 0.48 mmoles) was dissolved in trifluoroacetic acid (450µl). To the resulting solution was added dimethylsulfate (57.1 mg, 0.452 mmoles) at ambient temperature and allowed to stand at ambient temperature for 68 hours, to produce an alkylation solution. A proton NMR spectrum of the alkylation solution revealed that complete alkylation had occurred. The alkylation solution was then evaporated to dryness and the resultant residue was twice dissolved in methanol and evaporated to dryness, to produce an amber oil weighing 306.8 mg. The oil was dissolved in methanol (500 µl) and stirred in an atmosphere of nitrogen at ambient temperature while zinc dust (63 mg, 0.98 mmoles) was added portionwise over two hours. After complete addition of the zinc dust, the resultant mixture was allowed to continue stirring for 21 hours. An aliquot of the mixture was then removed for analysis by thin layer chromatography (silica gel; 3% methanol/dichloromethane as eluant) which revealed highly selective conversion to 3-amino-2-chloro-4-methylpyridine had occurred. The mixture was then filtered and the filtrate was concentrated to produce a dry residue. The residue was partitioned between ether and water (2 ml each). The layers were separated and the aqueous phase was extracted three additional times with 2 ml portions of ether. The ethereal extracts were combined and dried with anhydrous sodium sulfate, filtered and concentrated at reduced pressure to give 3-amino-2-chloro-4-methylpyridine as an oil weighing 66.6 mg. The oil solidified on standing. Product identity is confirmed by comparison of its proton NMR and TLC chromatographic properties (5% ethylacetate/dichloromethane, R_{f}=0.45; 3% methanol/dichloromethane, R_{f}=0.70) with those of an authentic sample.

## Claims

1. A method for the preparation of a compound of the formula 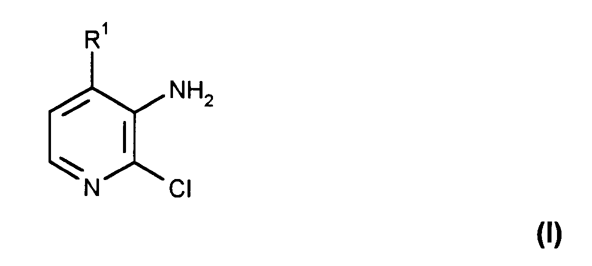 wherein R¹ is a linear, branched or cyclic hydrocarbon of from one to eight carbon atoms, optionally substituted with one or more electron stabilizing groups, which comprises
alkylating a compound of formula 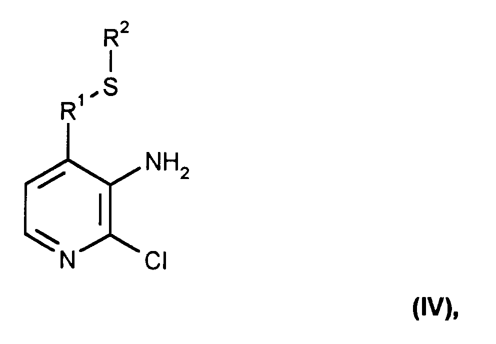 wherein
R¹ and R² are each a linear, branched or cyclic hydrocarbon of from one to eight carbon atoms; or
R¹ is a linear, branched or cyclic hydrocarbon of from one to eight carbon atoms, and R² is an aromatic or heteroaromatic group, such as phenyl, naphthyl, benzothiazolyl, benzoxazolyl, benzimidazolyl, pyridyl, furyl or thienyl; or
R¹ is a linear, branched or cyclic hydrocarbon of from one to eight carbon atoms, and R² is a linear, branched or cyclic hydrocarbon of from one to eight carbon atoms, wherein the carbon atom in R² bonded to the sulfur atom has no hydrogen bonded to it; or
R² is a linear, branched or cyclic hydrocarbon of from one to eight carbon atoms, and R¹ is a linear, branched or cyclic hydrocarbon of from one to eight carbon atoms optionally substituted with one or more electron stabilizing groups separated from the sulfur atom by one saturated carbon atom substituted with at least one hydrogen atom; or
R1 and R2 are each a linear or branched hydrocarbon of from one to eight carbon atoms, joined together with the sulfur atom to form a ring of from 5 to 8 members, wherein the ring can optionally be substituted with one or more electron stabilizing groups separated from the sulfur atom by one saturated carbon atom substituted with at least one hydrogen atom,
in the presence of an alkylating agent at a temperature of from 20°C to 100°C, for 1 to 140 hours, to produce a sulfonium salt and then contacting the sulfonium salt so produced with zinc at a temperature of from 0°C to 50°C, for 1 to 24 hours, to produce the compound of formula I.

2. A method for the preparation of a compound of formula IV, defined in claim 1, which comprises heating a compound of formula III 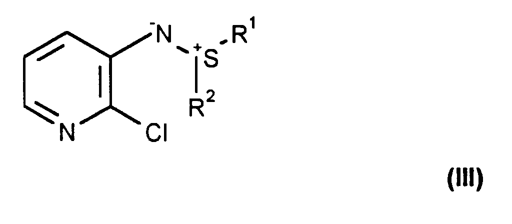 wherein R¹ and R² are as defined in claim 1, in the presence of: (i) triethylamine, methanesulfonic acid, and an organic solvent; or (ii) an organic solvent and a phenol of the formula: 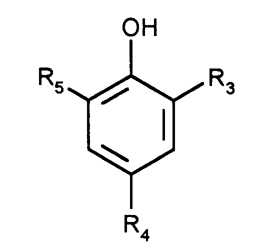
wherein R₃, R₄ and R₅ can each be H, C₁-C₈ straight chain or branched alkyl, Cl, Br or CH₂SR_{2,} wherein R₂ is as defined above;
R₄ can be -C(CH₃)₂-A, 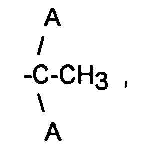 OR_{6,} wherein R₆ is C₁-C₈ straight chain alkyl, -[CH₂CH₂0]ₙ-CH₂CH₂OH and n is a number from 0 to 20, and A has the formula: 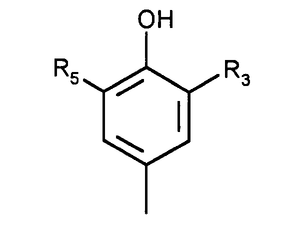
R₅ can be -CH₂-B, wherein B has the formula 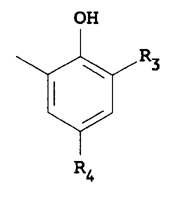
wherein R₃ is as defined above and R₄ can be as defined above, provided that it does not contain the group A,
or a phenol of the formula 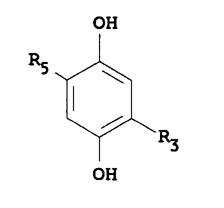
wherein R₃ is as defined above and R₅ is H or R₃,_{,}
at a temperature ranging from 60°C to 110°C, for 3 to 24 hours.

3. A method for the preparation of a compound of formula I, defined in claim 1, which comprises
heating the compound of formula III, defined in claim 2
in the presence of: (i) triethylamine, methanesulfonic acid, and an organic solvent; or (ii) an organic solvent and a phenol of the formula 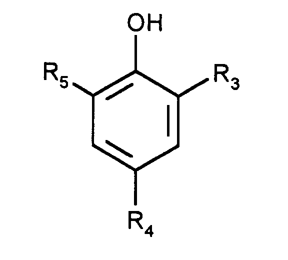
wherein R₃, R₄ and R₅ can each be H, C₁-C₈ straight chain or branched alkyl, Cl, Br or CH₂SR_{2,} wherein R₂ is as defined above;
R₄ can be -C(CH₃)₂-A, 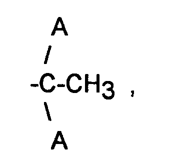 OR_{6,} wherein R₆ is C₁-C₈ straight chain alkyl, -[CH₂CH₂0]ₙ-CH₂CH₂OH and n is number from 0 to 20, and A has the formula 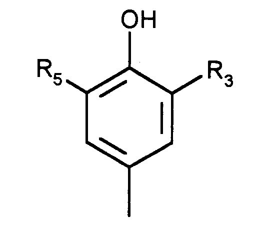
R₅ can be -CH₂-B, wherein B has the formula 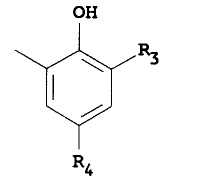
wherein R₃ is as defined above and R₄ can be as defined above, provided that it does not contain the group A,
or a phenol of the formula 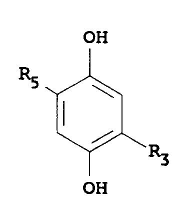
wherein R₃ is as defined above and R₅ is H or R_{3,}
at a temperature ranging from 60°C to 110°C, for 3 to 24 hours, to produce a compound of the formula 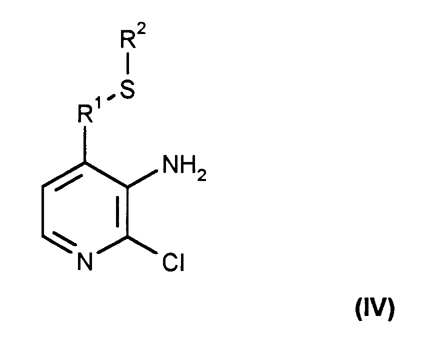 and either
heating the compound IV in the presence of ethanol and Raney nickel, at a temperature ranging from 25°C to 80°C, for 1 to 24 hours, to produce the compound of formula I, or
alkylating the compound IV in the presence of an alkylating agent at a temperature of from 20°C to 100°C, for 1 to 140 hours, to produce a sulfonium salt and then contacting the sulfonium salt so produced with zinc at a temperature of from 0°C to 50°C, for 1 to 24 hours, to produce the compound of formula I.

4. A method as recited in any one of claims 1 to 3 wherein R¹ and R² are each a linear, branched or cyclic hydrocarbon of from one to eight carbon atoms.

5. A method as recited in claim 4 wherein R¹ is a linear hydrocarbon of from one to four carbons and R² is a linear, branched or cyclic hydrocarbon of from one to eight carbon atoms.

6. A method as recited in claim 5 wherein R¹ is methyl or ethyl and R² is methyl, phenyl or 2-benzothiazolyl.

7. A method as recited in claim 2 or 3 wherein the phenol is selected from the group consisting of 2-t-butyl-4-methylphenol, 2,6-di-t-butyl-4-methylphenol or 2-t-butyl-4-methyl-6-methylthio-methylphenol.

8. A method as recited in claim 7 wherein the phenol is 2-t-butyl-4-methylphenol.

9. A method as recited in any one of claims 1 to 3 wherein the one or more electron stabilizing groups are selected from a group consisting of: C₆-C₁₀ aryl, C₁-C₈ carboalkoxy, carboxyamido, cyano, C₁-C₁₀ acyl (derived from aromatic or alkyl carboxylic acids), nitro, C₁-C₈ alkoxy, phosphoryl, C₁-C₈ alkyl, and C₆-C₁₀ arylthio, arylsulfinyl and arylsulfonyl.

10. A method as recited in claim 2 or 3 wherein the compound of formula III is heated in the presence of triethylamine, methanesulfonic acid and an organic solvent.

11. A method as recited in claim 10 wherein the organic solvent is toluene.

12. A method as recited in claim 2 or 3 wherein the compound of formula III is heated in the presence of 2-t-butyl-4-methylphenol and an organic solvent.

13. A method as recited in claim 12 wherein the organic solvent is toluene.

14. A method as recited in claim 1 or 3 wherein the alkylating agent is selected from the group consisting of methyl iodide, dimethylsulfate, allyl chloride, benzyl chloride, benzyl bromide, methyl chloride or chloroacetic acid.

15. A method as recited in claim 14 wherein the alkylating agent is dimethylsulfate.

## Patentansprüche

1. Verfahren zur Herstellung einer Verbindung der Formel 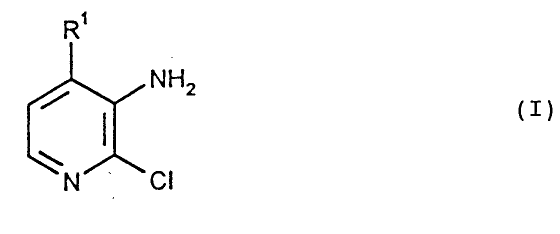 worin
R¹ einen geradkettigen, verzweigtkettigen oder cyclischen, gegebenenfalls mit einer oder mehreren elektronenstabilisierenden Gruppen substituierten Kohlenwasserstoff mit einem bis acht Kohlenstoffatomen bedeutet, umfassend
die Alkylierung einer Verbindung der Formel 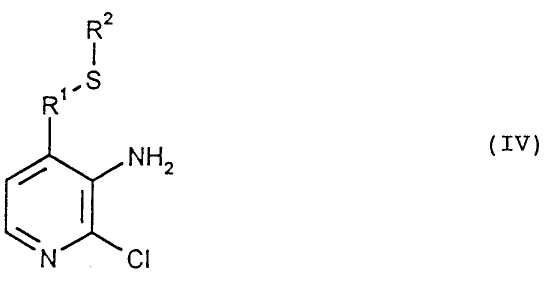 mit
jedem von R¹ und R² in der Bedeutung eines geradkettigen, verzweigtkettigen oder cyclischen Kohlenwasserstoffes mit einem bis acht Kohlenstoffatomen; oder
R¹ in der Bedeutung eines geradkettigen, verzweigtkettigen oder cyclischen Kohlenwasserstoffes mit einem bis acht Kohlenstoffatomen und R² in der Bedeutung eines aromatischen oder heteroaromatischen Rests wie Phenyl, Naphthyl, Benzothiazolyl, Benzoxazolyl, Benzimidazolyl, Pyridyl, Furyl oder Thienyl; oder
R¹ in der Bedeutung eines geradkettigen, verzweigtkettigen oder cyclischen Kohlenwasserstoffes mit einem bis acht Kohlenstoffatomen und R² in der Bedeutung eines geradkettigen, verzweigtkettigen oder cyclischen Kohlenwasserstoffes mit einem bis acht Kohlenstoffatomen bedeutet, wobei in R² das mit dem Schwefelatom verbundene Kohlenstoffatom kein damit verbundenes Wasserstoff aufweist; oder
R² in der Bedeutung eines geradkettigen, verzweigtkettigen oder cyclischen Kohlenwasserstoffes mit einem bis acht Kohlenstoffatomen und R¹ in der Bedeutung eines geradkettigen, verzweigtkettigen oder cyclischen Kohlenwasserstoffes mit einem bis acht Kohlenstoffatomen, das gegebenenfalls mit einer oder mehreren elektronenstabilisierenden Gruppen substituiert ist, die vom Schwefelatom durch ein mit mindestens einem Wasserstoffatom substituiertes gesättigtes Kohlenstoffatom getrennt sind; oder
jedem von R¹ und R² in der Bedeutung eines geradkettigen oder verzweigtkettigen Kohlenwasserstoffes mit einem bis acht Kohlenstoffatomen, die miteinander und mit dem Schwefelatom zur Bildung eines Ringes mit 5 bis 8 Gliedern verbunden sind, wobei der Ring gegebenenfalls mit einer oder mehreren elektronenstabilisierenden Gruppen substituiert sein kann, die vom Schwefelatom durch ein mit mindestens einem Wasserstoffatom substituiertes gesättigtes Kohlenstoffatom getrennt sind,
in Gegenwart eines Alkylierungsmittels bei einer Temperatur von 20°C bis 100°C während 1 bis 140 Stunden zur Produktion eines Sulfoniumsalzes, und danach die Kontaktierung des so produzierten Sulfoniumsalzes mit Zink bei einer Temperatur von 0°C bis 50°C während 1 bis 24 Stunden zur Produktion der Verbindung der Formel I.

2. Verfahren zur Herstellung einer Verbindung der im Anspruch 1 definierten Formel IV, umfassend die Erhitzung einer Verbindung der Formel III 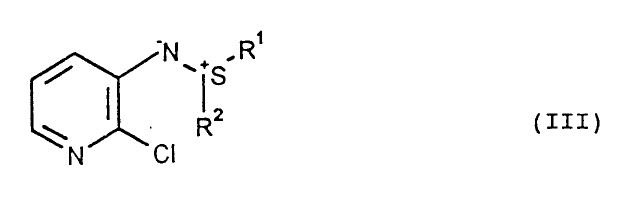 worin R¹ und R² wie im Anspruch 1 definiert sind, in Gegenwart von: (i) Triethylamin, Methansulfonsäure und eines organischen Lösungsmittels; oder (ii) eines organischen Lösungsmittels und eines Phenols der Formel: 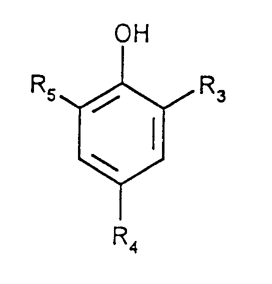 worin
jeder von R₃, R₄ und R₅ die Bedeutung H, geradkettiges oder verzweigtkettiges C₁-C₈-Alkyl, Cl, Br oder CH₂SR₂ mit R₂ wie im vorstehenden definiert haben kann;
R₄ die Bedeutung -C(CH₃)₂-A, 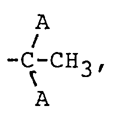 OR₆ haben kann, worin
R₆ geradkettiges C₁-C₈-Alkyl, -[CH₂CH₂O]ₙ-CH₂CH₂OH bedeutet und n eine natürliche Zahl von 0 bis 20 ist, und A der Formel 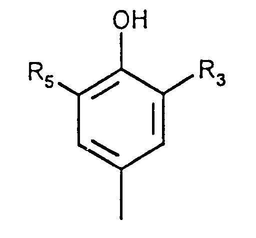 entspricht,
R₅ die Bedeutung -CH₂-B haben kann, worin B der Formel 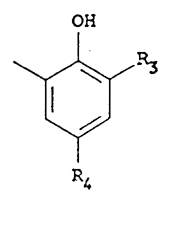 entspricht, worin R₃ die im vorstehenden definierte Bedeutung hat und R₄ die im vorstehenden definierte Bedeutung haben kann, vorausgesetzt, dass es den Rest A nicht enthält,
oder ein Phenol der Formel 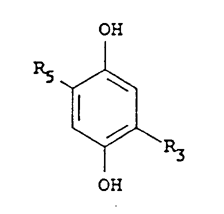
worin R₃ die im vorstehenden definierte Bedeutung und R₅ die Bedeutung H oder R₃ hat,
bei einer Temperatur im Bereich von 60°C bis 110°C während 3 bis 24 Stunden.

3. Verfahren zur Herstellung einer Verbindung der im Anspruch 1 definierten Formel I, umfassend
die Erhitzung der im Anspruch 2 definierten Verbindung der Formel III
in Gegenwart von: (i) Triethylamin, Methansulfonsäure und eines organischen Lösungsmittels; oder (ii) eines organischen Lösungsmittels und eines Phenols der Formel: 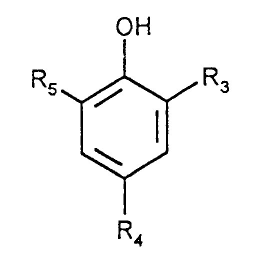 worin
jeder von R₃, R₄ und R₅ die Bedeutung H, geradkettiges oder verzweigtkettiges C₁-C₈-Alkyl, Cl, Br oder CH₂SR₂ mit R₂ wie im vorstehenden definiert haben kann;
R₄ die Bedeutung -C(CH₃)₂-A, 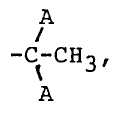 OR₆ haben kann, worin
R₆ geradkettiges C₁-C₈-Alkyl, -[CH₂CH₂O]ₙ-CH₂CH₂OH bedeutet und n eine natürliche Zahl von 0 bis 20 ist, und A der Formel 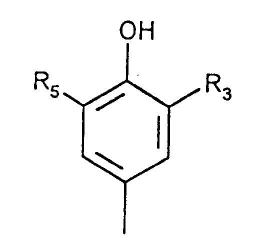 entspricht,
R₅ die Bedeutung -CH₂-B haben kann, worin B der Formel 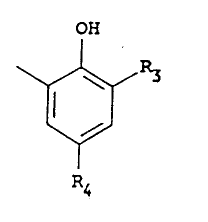 entspricht, worin R₃ die im vorstehenden definierte Bedeutung hat und R₄ die im vorstehenden definierte Bedeutung haben kann, vorausgesetzt, dass es den Rest A nicht enthält,
oder ein Phenol der Formel 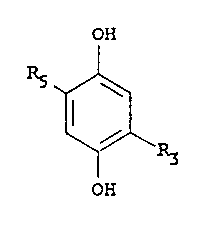
worin R₃ die im vorstehenden definierte Bedeutung und R₅ die Bedeutung H oder R₃ hat,
bei einer Temperatur im Bereich von 60°C bis 110°C während 3 bis 24 Stunden zur Produktion der Verbindung der Formel 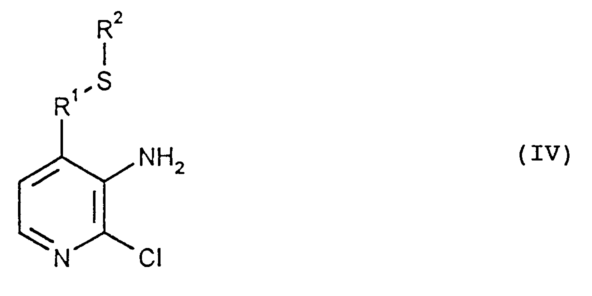 und
entweder die Erhitzung der Verbindung IV in Gegenwart von Ethanol und Raney-Nickel bei einer Temperatur im Bereich von 25°C bis 80°C während 1 bis 24 Stunden zur Produktion der Verbindung der Formel I,
oder die Alkylierung der Verbindung IV in Gegenwart eines Alkylierungsmittels bei einer Temperatur von 20°C bis 100°C während 1 bis 140 Stunden zur Produktion eines Sulfoniumsalzes, und danach die Kontaktierung des so produzierten Sulfoniumsalzes mit Zink bei einer Temperatur von 0°C bis 50°C während 1 bis 24 Stunden zur Produktion der Verbindung der Formel I.

4. Verfahren nach einem der Ansprüche 1 bis 3, bei welchem jeder von R¹ und R² einen geradkettigen, verzweigtkettigen oder cyclischen Kohlenwasserstoff mit einem bis acht Kohlenstoffatomen bedeutet.

5. Verfahren nach Anspruch 4, bei welchem R¹ einen geradkettigen Kohlenwasserstoff mit einem bis vier Kohlenstoffatomen und R² einen geradkettigen, verzweigtkettigen oder cyclischen Kohlenwasserstoff mit einem bis acht Kohlenstoffatomen bedeutet.

6. Verfahren nach Anspruch 5 mit R¹ in der Bedeutung Methyl oder Ethyl und R² in der Bedeutung Methyl, Phenyl oder 2-Benzothiazolyl.

7. Verfahren nach einem der Ansprüche 2 oder 3, bei welchem das Phenol aus der von 2-*tert*-Butyl-4-methylphenol, 2,6-Di-*tert*-butyl-4-methylphenol oder 2-*tert*-Butyl-4-methyl-6-methylthio-methylphenol gebildeten Gruppe ausgewählt ist.

8. Verfahren nach Anspruch 7, bei welchem das Phenol 2-*tert*-Butyl-4-methylphenol ist.

9. Verfahren nach einem der Ansprüche 1 bis 3, bei welchem die eine oder die mehreren elektronenstabilisierenden Gruppen aus der von C₆-C₁₀-Aryl, C₁-C₈-Carboalkoxy, Carboxyamido, Cyano, (von aromatischen oder Alkylcarbonsäuren abgeleitetes) C₁-C₁₀-Acyl, Nitro, C₁-C₈-Alkoxy, Phosphoryl, C₁-C₈-Alkyl und C₆-C₁₀-Arylthio, Arylsulfinyl und Arylsulfonyl gebildeten Gruppe ausgewählt ist/sind.

10. Verfahren nach einem der Ansprüche 2 oder 3, bei welchem die Verbindung der Formel III in Gegenwart von Triethylamin, Methansulfonsäure und eines organischen Lösungsmittels erhitzt wird.

11. Verfahren nach Anspruch 10, bei welchem das organische Lösungsmittel Toluol ist.

12. Verfahren nach einem der Ansprüche 2 oder 3, bei welchem die Verbindung der Formel III in Gegenwart von 2-*tert*-Butyl-4-methylphenol und eines organischen Lösungsmittels erhitzt wird.

13. Verfahren nach Anspruch 12, bei welchem das organische Lösungsmittel Toluol ist.

14. Verfahren nach einem der Ansprüche 1 oder 3, bei welchem das Alkylierungsmittel aus der von Methyliodid, Dimethylsulfat, Allylchlorid, Benzylchlorid, Benzylbromid, Methylchlorid oder Chloressigsäure gebildeten Gruppe ausgewählt ist.

15. Verfahren nach Anspruch 14, bei welchem das Alkylierungsmittel Dimethylsulfat ist.

## Revendications

1. Procédé de préparation d'un composé de formule 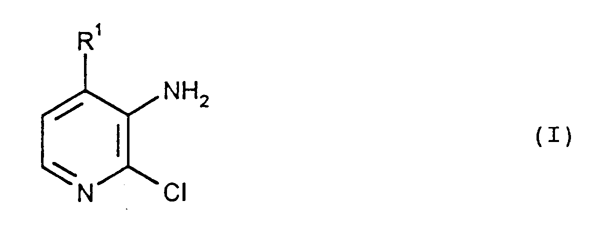 dans laquelle
R¹ représente un hydrocarbure linéaire, ramifié ou cyclique comportant un à huit atomes de carbone, facultativement substitué par un ou plusieurs groupes stabilisateurs d'électrons, comportant
l'alkylation d'un composé de formule 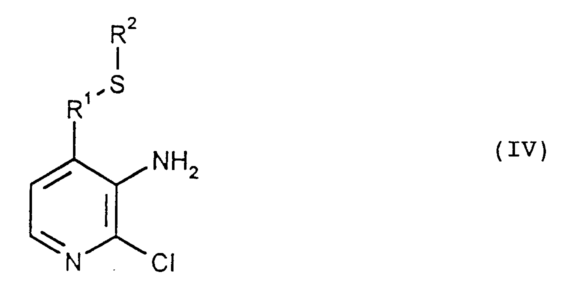 dans laquelle
chacun de R¹ et R² représente un hydrocarbure linéaire, ramifié ou cyclique comportant un à huit atomes de carbone; ou bien
R¹ représente un hydrocarbure linéaire, ramifié ou cyclique comportant un à huit atomes de carbone et R² représente un groupement aromatique hétéroaromatique tel que phényle, naphthyle, benzothiazolyle, benzoxazolyle, benzimidazolyle, pyridyle, furyle ou thiényle; ou bien
R¹ représente un hydrocarbure linéaire, ramifié ou cyclique comportant un à huit atomes de carbone et R² représente un hydrocarbure linéaire, ramifié ou cyclique comportant un à huit atomes de carbone, l'atome de carbone qui dans R² représente lié à l'atome de soufre ne comportant aucun hydrogène qui lui soit lié; ou bien
R² représente un hydrocarbure linéaire, ramifié ou cyclique comportant un à huit atomes de carbone et R¹ représente un hydrocarbure linéaire, ramifié ou cyclique comportant un à huit atomes de carbone, facultativement substitué par un ou plusieurs groupes stabilisateurs d'électrons séparés de l'atome de soufre par un atome de carbone saturé substitué par au moins un atome d'hydrogène; ou bien
chacun de R¹ et R² représente un hydrocarbure linéaire, ramifié ou cyclique comportant un à huit atomes de carbone reliés ensemble et avec l'atome de soufre de façon à former un anneau de 5 à 8 éléments, l'anneau pouvant être facultativement substitué par un ou plusieurs groupes stabilisateurs d'électrons séparés de l'atome de soufre par un atome de carbone saturé substitué par au moins un atome d'hydrogène;
en présence d'un agent d'alkylation à une température de 20°C à 100°C pendant 1 à 140 heures de façon à produire un sel de sulfonium, et ensuite la mise en contact du sel de sulfonium ainsi produit avec du zinc à une température de 0°C à 50°C pendant 1 à 24 heures de façon à produire le composé de formule I.

2. Procédé de préparation d'un composé de formule IV défini à la revendication 1, comportant le chauffage d'un composé de formule III 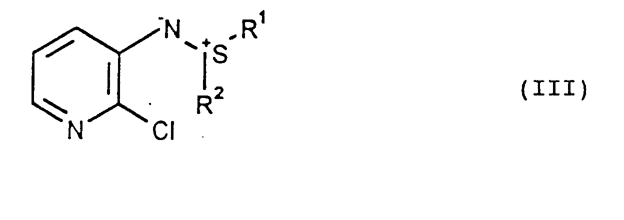 dans laquelle R¹ et R² sont définis comme à la revendication 1, en présence: (i) de triéthylamine, d'acide méthanesulfonique et d'un solvant organique; ou bien (ii) d'un solvant organique et d'un phénol de formule: 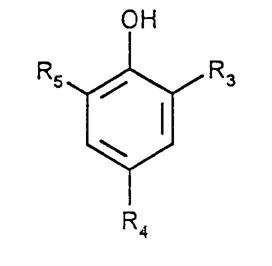 dans laquelle
chacun de R₃, R₄ et R₅ peut représenter H, un C₁-C₈-alkyle linéaire ou ramifié, Cl, Br ou CH₂SR₂ avec R₂ défini comme ci-dessus;
R₄ peut représenter -C(CH₃)₂-A, 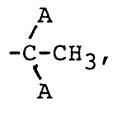 OR₆ dans lesquels
R₆ représente un C₁-C₈-alkyle linéaire, -[CH₂CH₂O]ₙ-CH₂CH₂OH, et n représente un nombre entier de 0 à 20, et A correspond à la formule 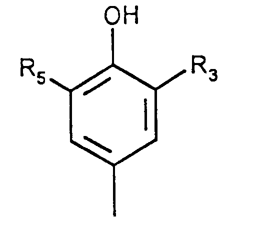
R₅ peut représenter -CH₂-B dans lequel B correspond à la formule 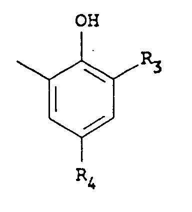
dans laquelle R₃ est défini comme ci-dessus et R₄ peut être comme défini ci-dessus sous réserve qu'il ne contienne pas le groupement A,
ou bien un phénol de formule 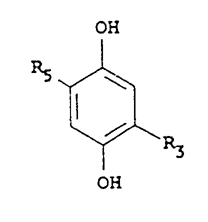
dans laquelle R₃ est défini comme ci-dessus et R₅ représente H ou R₃,
à une température allant de 60°C à 110°C pendant 3 à 24 heures.

3. Procédé de préparation d'un composé de la formule I définie à la revendication 1, comportant
le chauffage du composé de formule III définie à la revendication 2
en présence: (i) de triéthylamine, d'acide méthanesulfonique et d'un solvant organique; ou bien (ii) d'un solvant organique et d'un phénol de formule: 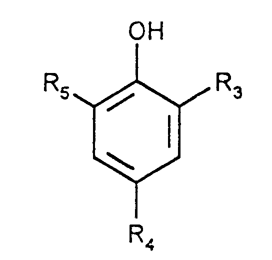 dans laquelle
chacun de R₃, R₄ et R₅ peut représenter H, un C₁-C₈-alkyle linéaire ou ramifié, Cl, Br ou CH₂SR₂ avec R₂ défini comme ci-dessus;
R₄ peut représenter -C(CH₃)₂-A, 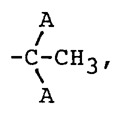 OR₆, dans lesquels
R₆ représente un C₁-C₈-alkyle linéaire, -[CH₂CH₂O]ₙ-CH₂CH₂OH, et n représente un nombre entier de 0 à 20, et A correspond à la formule 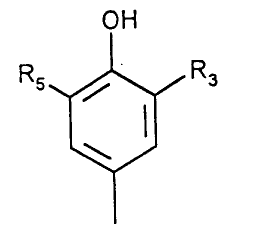
R₅ peut représenter -CH₂-B dans lequel B correspond à la formule 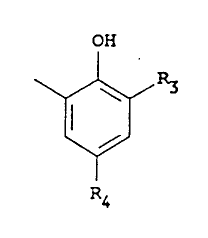
dans laquelle R₃ est défini comme ci-dessus et R₄ peut être comme défini ci-dessus sous réserve qu'il ne contienne pas le groupement A,
ou bien un phénol de formule 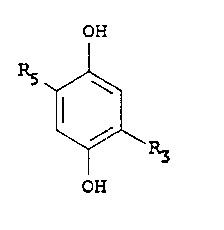
dans laquelle R₃ est défini comme ci-dessus et R₅ représente H ou R₃,
à une température allant de 60°C à 110°C pendant 3 à 24 heures, de façon à produire le composé de formule 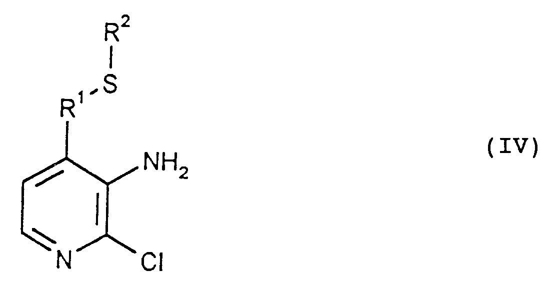 et
ou bien le chauffage du composé IV en présence d'éthanol et de nickel de Raney à une température allant de 25°C à 80°C pendant 1 à 24 heures de façon à produire le composé de formule I,
ou bien l'alkylation du composé IV en présence d'un agent d'alkylation à une température de 20°C à 100°C pendant 1 à 140 heures de façon à produire un sel de sulfonium, et ensuite la mise en contact du sel de sulfonium ainsi produit avec du zinc à une température de 0°C à 50°C pendant 1 à 24 heures de façon à produire le composé de formule I.

4. Procédé selon l'une des revendications 1 à 3, dans lequel chacun de R¹ et R² représente un hydrocarbure linéaire, ramifié ou cyclique comportant un à huit atomes de carbone.

5. Procédé selon la revendication 4, dans lequel R¹ représente un hydrocarbure linéaire comportant un à quatre atomes de carbone et R² représente un hydrocarbure linéaire, ramifié ou cyclique comportant un à huit atomes de carbone.

6. Procédé selon la revendication 5 avec R¹ représentant méthyle ou éthyle et R² représentant méthyle, phényle ou 2-benzothiazolyle.

7. Procédé selon l'une des revendications 2 ou 3, dans lequel le phénol est choisi parmi le groupe formé de 2-*tert*-butyl-4-méthylphénol, 2,6-di-*tert*-butyl-4-méthylphénol ou 2-*tert*-butyl-4-méthyl-6-méthylthio-méthylphénol.

8. Procédé selon la revendication 7, dans lequel le phénol est le 2-*tert*-butyl-4-méthylphénol.

9. Procédé selon l'une des revendications 1 à 3, dans lequel ledit ou lesdits groupes stabilisateurs d'électrons sont choisis parmi le groupe formé de C₆-C₁₀-aryle, C₁-C₈-carboalkoxy, carboxyamido, cyano, C₁-C₁₀-acyle (dérivé d'acides aromatiques ou alkylcarboxyliques), nitro, C₁-C₈-alkoxy, phosphoryle, C₁-C₈-alkyle et C₆-C₁₀-arylthio, arylsulfinyle et arylsulfonyle.

10. Procédé selon l'une des revendications 2 ou 3, dans lequel le composé de formule III est chauffé en présence de triéthylamine, d'acide méthane-sulfonique et d'un solvant organique.

11. Procédé selon la revendication 10, dans lequel le solvant organique est le toluène.

12. Procédé selon l'une des revendications 2 ou 3, dans lequel le composé de formule III est chauffé en présence de 2-*tert*-butyl-4-méthylphénol et d'un solvant organique.

13. Procédé selon la revendication 10, dans lequel le solvant organique est le toluène.

14. Procédé selon l'une des revendications 1 ou 3, dans lequel l'agent d'alkylation est choisi parmi le groupe formé d'iodure de méthyle, sulfate de diméthyle, chlorure d'allyle, chlorure de benzyle, bromure de benzyle, chlorure de méthyle ou acide chloracétique.

15. Procédé selon la revendication 10, dans lequel l'agent d'alkylation est le sulfate de diméthyle.
